# EUROPEAN PATENT SPECIFICATION

(11) **EP 2 101 723 B1**
(45) Date of publication and mention of the grant of the patent: **13.08.2014**
(21) Application number: 07851480.9
(22) Date of filing: 13.12.2007
(51) Int. Cl.: A61K 8/97, A61Q 19/08

(54) **COSMETIC COMPOSITION FOR PROTECTING SKIN AGAINST UV LIGHT AND WRINKLE IMPROVEMENT CONTAINING THE EXTRACT OF MAGNOLIA SIEBOLDII FLOWER EXTRACTS**
KOSMETISCHE ZUSAMMENSETZUNG ZUM SCHUTZ DER HAUT VOR UV-LICHT UND ZUR LINDERUNG VON FALTEN MIT DEM EXTRAKT VON MAGNOLIA SIEBOLDII BLÜTEN
COMPOSITION COSMÉTIQUE POUR PROTÉGER LA PEAU CONTRE LA LUMIÈRE ULTRAVIOLETTE ET ATTÉNUER LES RIDES, QUI CONTIENT L'EXTRAIT DE FLEUR DE MAGNOLIA SIEBOLDII

(30) Priority: 08.01.2007 KR 20070001833
(43) Date of publication of application: 23.09.2009
(73) Proprietor: Coreana Cosmetics Co., Ltd., 330-833 Cheonan-shi, Chungcheongnam-do (KR)
(72) Inventor: LEE, Ghang Tai, Cheonan-Si Chungcheongnam-do 330-835 (KR); LEE, Jung Noh, Chungcheongnam-do 339-752 (KR); YOU, Young Kyoung, Cheongju Si Chungcheongbuk-do 360-813 (KR); LEE, Seung Ji, CheonanSi Chungcheongnam-do 330-786 (KR); LEE, Kun Kook, Seoul 138-788 (KR)
(74) Representative: Catherine, Alain
(86) International application number: PCT/KR2007/006509
(87) International publication number: WO 2008/084923

(56) References cited:
- FR-A1- 2 808 190
- JP-A- 2006 328 048
- KR-A- 19980 025 843
- KR-A- 20060 058 530
- US-B1- 6 464 839
- "An aging preventive agent caused by sunlight composed of an extract of Magnolia obovata, optionally further containing up to nine plant extracts used for foods, drink preparations and cosmetics" DERWENT, 10 April 2002 (2002-04-10), XP002312496
- DATABASE WPI Week 200042 Thomson Scientific, London, GB; AN 2000-476612 XP002599271 & CN 1 244 572 A (ZHANG G) 16 February 2000 (2000-02-16)
- DATABASE [Online] 28 July 1997 DELICONSTANTINOS ET AL: 'Inhibition of ultraviolet B-induced skin erythema by N-nitro-L-arginine and N-monomethyl-L-arginine', XP002093162 Retrieved from CHEMICAL
- HEE-JUHN,WON-TAE,PURUSOTAM,TSUNEO: "syringin-4-o-beta-glucoside,a new phenylpropanoid glycoside,and costunolide, a nitric oxide synthase inhibitor,from the stem bark of Magnolia Sieboldii", J. NAT. PROD., [Online] vol. 59, 16 May 1996 (1996-05-16), pages 1128-1130,

## Description

### Technical Field

The present invention relates to a cosmetic composition containing an extract of *Magnolia sieboldii* as an active ingredient, and more particularly to a cosmetic composition for protecting skin against UV light and wrinkle improvement(reducing skin wrinkles) containing an extract of *Magnolia sieboldii,* and thus serves to protect DNA and skin cells from UV light damage, promote the biosynthesis of collagen and inhibit the biosynthesis of collagenases.

### Background Art

In most people, the skin readily copes with sunlight in the growth stage, but the skin tissue changes gradually as the age increases, and thus, various events, including skin roughness, wrinkles, coloring spots, bad complexions, diarrhea, capillary vasodilation, black spots, purpura, easy bruises, contraction, fibrosis, discolored sites, and in severe cases, premalignant tumors and malignant tumors, occur due to sunlight.

UV light is divided into UVA, UVB and UVC ranges, in which only UVA and UVB reach the earth surface, and UVC is filtered out by the ozone layer. UVB has the greatest effect on the skin and penetrates the epidermis to damage skin cells. UVA penetrates the skin dermis to damage collagen synthesis associated skin cells. These UV lights directly damage the skin, or indirectly damage the skin by generating active oxygen. The effects of UV lights on skin cells are as follows. When keratinocytes among skin cells are irradiated with UV lights, they secrete inflammatory cytokines and induce oxidative stress, so that various kinds of cellular protein kinases and phospholipases are activated. These activated enzymes hydrolyze cell membrane lipids to secrete arachidonic acid. The secreted arachidonic acid is converted into prostaglandin E2 (PEG2) by the action of cyclooxygenase. The formed arachidonic acid and PGE2 cause various inflammatory reactions, including erythema, in skin in cooperation with cytokines, thus promoting skin aging.

Also, fibroblasts among skin cells are activated by UV radiation according to a process similar to the above-described keratinocyte activation. In some cases, with an increase in the amount of UV lights, fibroblasts are killed, or if DNA are damaged, it results in causing skin cancer and promoting skin aging. That is, because UV lights can cause skin erythema, immune suppression, wrinkle production and, ultimately, skin aging and skin cancer, the blockage and prevention of UV irradiation are important for the prevention of skin cancer and the delay of skin aging.

These days, due to the breakdown of the ozone layer resulting from air pollution and environmental contamination, the amount of UV lights reaching the earth surface increases, and the wavelength range of UV lights also change, so that skin aging and skin cancers increase and are expected to further increase in future. Accordingly, it is very important in the cosmetic field to protect the skin from UV lights, and many cosmetic manufacturing companies have conducted many studies to develop the related products. In currently used methods, simple UV blockers or absorbers are used to prevent the skin from being damaged by UV lights, and recently, it was reported that these UV blockers themselves can induce mutations to cause skin cancer.

Because these skin damages caused by UV lights are ultimately associated with skin aging, a method capable of preventing these skin damages is a concern in the cosmetic field. Methods for preventing skin damage caused by UV lights include methods of suppressing active oxygen or the production thereof, and another method comprises blocking UV lights. Most UV light-associated products are characterized in that they block UV lights, and recently, methods of using natural substances to suppress active oxygen caused by UV light have been frequently used.

### Disclosure of Invention

### Technical Problem

The present inventors have made many efforts to find a physiologically active plant substance, which does not show only simple blocking action, as the prior UV blockers do, but shows no toxicity and can fundamentally prevent skin cells from being damaged by UV lights, thus fundamentally preventing skin aging and skin cancer. As a result, the present inventors have found that an extract of *Magnolia sieboldii* has an excellent effect of preventing the skin from being damaged by UV lights, thereby completing the present invention.

### Technical Solution

Therefore, it is an object of the present invention to provide a cosmetic composition for preventing photoaging containg an extract of *Magnolia sieboldii* as an active ingredient.

Another object of the present invention is to provide a cosmetic composition for wrinkle improvement(reducing skin wrinkles) containing an extract of *Magnolia sieboldii* as an active ingredient.

Still another object of the present invention is to provide a make-up method comprising applying to the human skin said cosmetic composition containing the *Magnolia sieboldii* extract.

Other objects and advantages of the present invention will become more apparent from the following detailed description and the appended claims.

### Advantageous Effects

The present invention provides a cosmetic composition for preventing photoaging containing an extract of *Magnolia sieboldii* as an active ingredient. In another aspect, the present invention provides a cosmetic composition for reducing skin wrinkles containing an extract of *Magnolia sieboldii* as an active ingredient. The *Magnolia sieboldii* extract according to the present invention can protect the skin from UV-light damage by inhibiting the skin cell death and skin cell DNA damage caused by UV lights and can reduce skin wrinkles by promoting collagen biosynthesis and suppressing the synthesis of collagenase. Also, the cosmetic compositions containing the *Magnolia sieboldii* extract of the present invention has an excellent effect of inhibiting skin damage caused by UV lights, and an excellent effect of reducing skin wrinkles.

### Best Mode for Carrying Out the Invention

According to one aspect of the present invention, there is provided a cosmetic composition for preventing photoaging containing an extract of *Magnolia sieboldii.*

According to another aspect of the present invention, there is provided a cosmetic composition for wrinkle improvement(reducing skin wrinkles) containing an extract of *Magnolia sieboldii.*

*Magnolia sieboldii,* which is the active ingredient of the cosmetic composition of the present invention, is a deciduous small tree belonging to the *Magnoliaceae* family, and the *Magnolia sieboldii* extract is obtained from the flower of *Magnolia sieboldii.*

*Magnolia sieboldii* is about 7 meters in height, and the leaves thereof are alternate, long oval-shaped and glassy. The white large flowers thereof open downward in May and June, and the fruits thereof are ripe in September. It is an ornamental plant, grows in the valleys of mountains and is distributed in all parts of Korea except for Hamgyeongbuk-do, Japanese and China.

The *Magnolia sieboldii* extract that is used in the present invention is obtained from the flower of a *Magnolia sieboldii* tree.

The *Magnolia sieboldii* extract that is used in the present invention can be obtained through various methods known in the art. Preferably, the *Magnolia sieboldii* extract can be obtained using, as an extraction solvent, (a) water, (b) an anhydrous or hydrous lower alcohol having 1-4 carbon atoms (e.g., methanol, ethanol, butanol, etc.), (c) acetone, (d) ethyl acetate, (e) chloroform, or (f) 1,3-butylene glycol. The extraction solvent is more preferably ethanol, and more preferably about 70% ethanol. *Magnolia sieboldii* that is used in the extraction process can be used after drying or without drying. Preferably, it is used after drying. The drying process can be natural drying or hot-air drying.

In one preferred embodiment of the present invention, a method for preparing the *Magnolia sieboldii* extract of the present invention comprises: adding hydrous ethanol to dried *Magnolia sieboldii,* extracting the *Magnolia sieboldii*-containing solution at room temperature for a few days, filtering the extracted solution, and concentrating the filtrate in a vacuum, thus obtaining a dried extract

Meanwhile, it will be obvious to those skilled in the art that, even when extraction solvents other than said extraction solvents are used, *Magnolia sieboldii* extracts showing substantially the same effects can be obtained.

Moreover, the scope of the *Magnolia sieboldii* extract of the present invention includes not only the extract obtained through the above-described extraction method, but also an extract obtained through a conventional purification process. Fractions obtained through various additional purification methods, for example, separation with an ultrafiltration membrane having a given molecular weight cut-off, separation by various chromatography systems (manufactured for separation according to size, charge, hydrophobicity or affinity), are also included in the scope of the *Magnolia sieboldii* extract of the present invention.

The *Magnolia sieboldii* extract of the present invention can be prepared in the form of powder through an additional process, such as vacuum distillation, freezing drying or spray drying.

The *Magnolia sieboldii* extract of the present invention has an activity of protecting the skin from skin damage caused by external factors such as UV lights.

UVB has the greatest effect on the skin and penetrates the epidermis to damage skin cells. UVA penetrates the skin dermis to damage collagen synthesis associated skin cells. These UV lights directly damage the skin, or indirectly damage the skin by generating active oxygen.

The external factors include UV lights, stresses and external harmful chemical substances. These factors generate free radicals (e.g., hydroxyl radicals, superoxide radicals, active oxygen and hydrogen peroxide) to increase the oxidative stress of the skin so as to cause inflammation, skin cell death and DNA damage of skin cell, it results in causing skin cancer and promoting skin aging.

As used herein, the term "skin damage means that the skin cannot perform the normal function thereof, it includes damage to skin constituents (collagen, elastin, etc.), damage to the function of skin cells, and the death of skin cells.

As used herein, the term "skin cell" is meant to include all cells present in the skin, preferably keratinocytes and fibroblasts.

As used herein, the term "protecting skin" is meant to include preventing damage to the skin and improving damaged skin.

Accordingly, as used herein, the term prevention of skin damage caused by UV lights and protecting skin from UV lights, that is, "photoaging prevention" has the same means as reducing skin wrinkles by suppressing the production of active oxygen, promoting collagen synthesis and suppressing the production of collagenase.

The *Magnolia sieboldii* extract of the present invention can remove the oxidative stress of the skin resulting from UV lights, to suppress skin cell death and prevent DNA damage, thus protecting the skin. In a long-term viewpoint, it can fundamentally prevent skin cancer development and skin aging and can effectively improve skin wrinkles.

The *Magnolia sieboldii* extract of the present invention can exhibit the effect thereof by locally applying or spraying it on the skin. Thus, according to one preferred embodiment of the present invention, the composition of the present invention can be prepared in the form of cosmetic compositions, including cream, milk lotion and skin lotion, or pharmaceutical compositions, including spray and ointment.

The content of the *Magnolia sieboldii* extract in the composition is 0.0001-20.0 wt%, and preferably 0.01-10 wt%, based on the total weight of the composition.

When the composition of the present invention is formulated into a cosmetic product, the cosmetic composition contains, in addition to the *Magnolia sieboldii* extract as an active ingredient, components which are conventionally used in cosmetic compositions, for example, conventional additives, such as an antioxidant, a stabilizer, a solubilizing agent, vitamins, a pigment and perfume, and carrier components.

The inventive cosmetic composition for skin protection can be prepared in any formulation, which is conventionally prepared in the art. For example, it can be formulated into solution, suspension, emulsion, paste, gel, cream, lotion, powder, soap, surfactant-containing cleansing, oil, powder foundation, emulsion foundation, wax foundation and spray, but the scope of the present invention is not limited thereto. More specifically, it can be prepared in the form of skin lotion, milk lotion, nutrition cream, massage cream, essence, eye cream, cleansing cream, cleansing foam, cleansing water, pack, spray or powder.

If the formulation of the present invention is paste, cream or gel, it may contain, as carrier components, animal oil, vegetable oil, wax, paraffin, starch, tragacanth, cellulose derivatives, polyethylene glycol, silicone, bentonite, silica, talc or zinc oxide.

If the formulation of the present invention is powder or spray, it may contain, as carrier components, lactose, talc, silica, aluminum hydroxide, calcium silicate or polyamide powder. Particularly, if it is spray, it may additionally contain a propellant, such as chlorofluorohydrocarbon, propane/butane or dimethyl ether.

If the formulation of the present invention is solution or emulsion, it may contain, as carrier components, a solvent, a solubilizing agent or an emulsifying agent, for example, water, ethanol, isopropanol, ethyl carbonate, ethyl acetate, benzyl alcohol, benzyl benzoate, propylene glycol, 1,3-butyl glycol oil, glycerol fatty ester, polyethylene glycol or sorbitan fatty acid ester.

If the formulation of the present invention is suspension, it may contain, as carrier components, a liquid diluent, such as water, ethanol or propylene glycol, and a suspending agent, such as ethoxylated isostearyl alcohol, polyoxyethylene sorbitol ester, polyoxyethylene sorbitan ester, microcrystalline cellulose, aluminum metahydroxide, bentonite, agar-agar or tragacanth.

If the formulation of the present invention is a surfactant-containing cleansing, it may contain, as carrier components, aliphatic alcohol sulfate, aliphatic alcohol ether sulfate, sulfosuccinic monoester, isethionate, imidazolium derivatives, methyl taurate, sarcosinate, fatty acid amide ether sulfate, alkyl amido betaine, aliphatic alcohol, fatty acid glyceride, fatty acid diethanolamide, vegetable oil, lanoline derivatives, or ethoxylated glycerol fatty acid ester.

In another aspect, the present invention provides a make-up method comprising applying to the human skin the inventive cosmetic composition containing the *Magnolia sieboldii* extract.

The make-up method of the present invention includes all make-up methods comprising applying the inventive cosmetic composition to the human skin. That is, all methods known in the art, comprising applying the cosmetic composition to the skin, are included in the cosmetic method of the present invention.

The cosmetic composition of the present invention can be used alone or in combination with other cosmetic compositions. Also, the inventive cosmetic composition having an excellent skin-protecting effect can be used according to any conventional method, and the number of use thereof can vary depending on the skin condition of users or tastes in users.

If the cosmetic composition of the present invention is soap, a surfactant-containing cleansing formulation or a cleansing formulation containing no surfactant, it can be wiped, removed or washed with water, after it is applied to the skin. Concrete examples of said soap include, but are not limited to, liquid soap, powder soap, solid soap and oil soap, examples of said surfactant-containing cleansing formulation include, but are not limited to, cleansing foam, cleansing water, cleansing towel and cleansing pack, and examples of said surfactant-not containing cleansing formulation include, but are not limited to, cleansing cream, cleansing water and cleansing gel.

The inventive make-up method comprising applying to the human skin the inventive cosmetic composition containing the *Magnolia sieboldii* extract is carried out, photoaging-preventing effects and skin wrinkle-reducing effects, including the protection of DNA and skin cells from UV light damage, the promotion of collagen biosynthesis and the inhibition of collagenase biosynthesis, can be obtained.

Hereinafter, the present invention will be described in further detail with reference to examples. It is to be understood, however, that these examples are illustrative only, and the scope of the present invention is not limited thereto.

### Preparation Example 1: Preparation of flower extract of Magnolia sieboldii

100 g of the dried flower of *Magnolia sieboldii* (purchased from Kyung Dong Market, Korea) was added to 1.0 L of 70% ethanol (ethanol: water = 7:3) and extracted at room temperature for 3 days. The extracted solution was filtered, and the filtrate was concentrated in a vacuum, thus obtaining a dried extract (extraction yield: 7.8 g/100 g). The dried extract was dissolved in 30% 1,3-butylene glycol solution to a concentration of 1% (w/v) and filtered. Then, the resulting extract was used in the experiment of the present invention.

### Preparation Examples 2: Preparation of whole plant extract of Magnolia sieboldii

100 g of the dried whole plant of *Magnolia sieboldii* (purchased from Kyung Dong Market, Korea) was added to 1.0 L of 70% ethanol (ethanol: water = 7:3) and extracted at room temperature for 3 days. The extracted solution was filtered, and the filtrate was concentrated in a vacuum, thus obtaining a dried extract (extraction yield: 7.0 g/100 g). The dried extract was dissolved in 30% 1,3-butylene glycol solution to a concentration of 1% (w/v) and filtered. Then, the resulting extract was used in the experiment of the present invention.

### Experimental Example 1: Inhibitory effect on keratinocyte cell death caused by UV lights

When keratinocytes are irradiated with UV rays, active oxygen is generated in the cells. In order to artificially induce this phenomenon, human normal keratinocytes, Hacat cells (Korea Cell Line Bank) were inoculated into each well of a 48-well microplate at a density of 1 x 10⁴ cells/well and cultured in DMEM medium (Sigma, USA) at 37 °C for 24 hours. The cultured cells were irradiated with UV ray (UVB), and then the *Magnolia sieboldii* extract of Preparation Example 1 was added to the cell culture in each well at concentrations of 0.1, 0.5, 1.0 and 2.0% (v/v). After 24 hours, the activity of the cells was analyzed by the MTT assay.

**Table 1**

| Concentration (v/v%) *Magnolia sieboldii* extract of Preparation Example 1 | Cell viability (%) | |
|---|---|---|
| | Cells not irradiated with UV light | Cells irradiated with UV light |
| 0.0 | 100.0 | 2.5 |
| 0.1 | 101.2 | 35.6 |
| 0.5 | 102.2 | 46.8 |
| 1.0 | 105.5 | 53.5 |
| 2.0 | 108.5 | 88.3 |

As can be seen in Table 1 above, the *Magnolia sieboldii* extract of the present invention concentration-dependently inhibited keratinocyte death caused by UV irradiation, and showed an excellent effect on the inhibition of keratinocyte death.

### Experimental Example 2: Inhibitory effect on fibroblast cell death caused by UV light s

When fibroblasts are irradiated with UV rays, active oxygen is generated to damage the cells. In order to artificially induce this phenomenon, human fibroblast cells (Korea Cell Line Bank) were inoculated into each well of a 48-well microplate at a density of 1 x 10⁴cells/well and cultured in DMEM medium (Sigma, USA) at 37°C for 24 hours. The cultured cells were irradiated with UV ray (UVA), and then the *Magnolia sieboldii* extract of Preparation Example 1 was added to the cell culture in each well at concentrations of 0.1, 0.5, 1.0 and 2.0% (v/v). After 24 hours, the activity of the cells was analyzed by the MTT assay.

**Table 2**

| Concentration (v/v%) of *Magnolia sieboldii* extract of Preparation Example 1 | Cell viability (%) | |
|---|---|---|
| | Cells not irradiated with UV light | Cells irradiated with UV light |
| 0.0 | 100.0 | 12.5 |
| 0.1 | 105.3 | 55.6 |
| 0.5 | 103.5 | 66.8 |
| 1.0 | 100.7 | 83.5 |
| 2.0 | 100.5 | 99.3 |

As can be seen in Table 2 above, the *Magnolia sieboldii* extract of the present invention concentration-dependently inhibited fibroblast death caused by UV irradiation, and showed an excellent effect on the inhibition of keratinocyte cell death.

### Experimental Example 3: Inhibitory effect on fibroblast DNA damage caused by UV lights

When fibroblasts are irradiated with UV rays, the DNA of the fibroblasts is damaged. The prevention of fibroblast DNA damage is an important factor in the prevention of skin cancer and the delay of aging. Accordingly, in order to examine whether the *Magnolia sieboldii* extract of the present invention has an effect of inhibiting the induction of keratinocyte DNA damage, the following test was carried out.

Fibroblasts (Korea Cell Line Bank) were inoculated into each well of a 24-well plate at a density of 5 x 10⁴ cells/well. The cells in each well were irradiated with UV light (UVA), and then the *Magnolia sieboldii* extract of Preparation Example 1 was added to the cell culture in each well at concentrations of 1.0 and 2.0% (v/v). After 24 hours, the cells were subjected to single-cell gel electrophoresis (Comet assay) (Examples 1 and 2). For comparison, a control group, which was not irradiated with UV and not treated with the *Magnolia sieboldii* extract, and an comparative example (Comparative Example 1), which was irradiated with UV light, but was not treated with the *Magnolia sieboldii* extract, were used. The tail length(□) of the cells was measured with an image analyzer (Komet, 3.1, Kinetic imaging, England), and based on the measurement results, the extent of damage to DNA was analyzed.

**Table 3**

| | Concentration (v/v%) of *Magnolia sieboldii* extract of Preparation Example 1 | Mean tail length (□) |
|---|---|---|
| Control group | - | 53.1 |
| Comparative Example 1 | 0 | 152.8 |
| Example 1 | 1.0 | 67.5 |
| Example 2 | 2.0 | 56.5 |

As can be seen in Table 3, the *Magnolia sieboldii* extract of the present invention concentration-dependently inhibited fibroblast DNA damage caused by UV light (see Comparative Example 1 and Examples 1 and 2). The results of Example 2, in which the cells were treated with 2 vol% of the *Magnolia sieboldii* extract of Preparation Example 1, were almost similar to those of the control group. This demonstrates that the *Magnolia sieboldii* extract of the present invention has a very excellent effect of inhibiting fibroblast DNA damage caused by UV light.

### Experimental Example 4: Effect of Magnolia sieboldii extract on inhibition of collagenase production

This Experimental example was carried out using the sample, obtained in Example, in the following manner. First, human normal fibroblasts were inoculated into each well of a 48-well microplate (Nunc, Denmark) at a density of 1 x 10⁶ cells/wells and cultured in DMEM medium (Sigma, USA) at 37 °C for 24 hours. Then, the culture medium was replaced with each of serum-free DMEM media containing the *Magnolia sieboldii* extract (obtained in each of Preparation Examples 1 and 2) at varying concentrations of 0.01, 0.1, 1.0,2.0 and 5.0% (v/v), and the cells were additionally cultured for 48 hours. For comparison, the cells were additionally cultured in serum-free medium containing no *Magnolia sieboldii* extract (Control Example 2). After the cells were cultured, the supernatant of each well of the plate was collected, and the production of collagenase in the supernatant was measured using a collagenase assay kit (Amersham, USA). The measurement results are shown in Table 4 below.

**Table 4**

| Effect of *Magnolia sieboldii* extract on inhibition of collagenase production | | | |
|---|---|---|---|
| | Flower extract (v/v%) of *Magnolia sieboldii* of Preparation Example 1 | Whole plant extract (v/v%) of *Magnolia sieboldii* of Preparation Example 2 | Production of collagenase |
| Control Example 2 | - | - | 2.358 |
| Example 3 | 0.01 | - | 2.058 |
| Example 4 | 0.10 | - | 1.944 |
| Example 5 | 1.00 | - | 1.356 |
| Example 6 | 2.00 | - | 1.325 |
| Example 7 | 5.00 | - | 1.365 |
| Example 8 | - | 0.01 | 2.301 |
| Example 9 | - | 0.10 | 2.213 |
| Example 10 | - | 1.00 | 2.015 |
| Example 11 | - | 2.00 | 1.989 |
| Example 12 | - | 5.00 | 1.825 |

As can be seen from the results in Table 4, the *Magnolia sieboldii* extract of the present invention inhibited the production of collagenase, unlike Control Example 2 which was not treated with anything. As can be seen from the results of Examples 3 to 7 and Examples 8 to 12, the *Magnolia sieboldii* extract of the present invention concentration-dependently inhibited the production of collagenase. Also, it could be observed that the flower extract of *Magnolia sieboldii* was superior to the whole plant extract of *Magnolia sieboldii* with respect to the inhibition of collagenase production.

### Experimental Example 5: Effect of Magnolia sieboldii extract on promotion of collagen synthesis

Human normal fibroblasts were inoculated into each well of a 48-well microplate at a density of 1 x 10⁶ cells and cultured in DMEM medium at 37 °C for 24 hours. Then, the culture medium was replaced with each of serum-free media containing the *Magnolia sieboldii* extract (obtained in each of Preparation Examples 1 and 2) at varying concentrations of 0.01, 0.10, 1.0, 2.0 and 5.0 v/v%, and then the cells were additionally cultured for 24 hours (Examples 13 to 22). For comparison, the cells were additionally cultured in serum-free DMEM medium containing no *Magnolia sieboldii* extract for 24 hours (Control Example 3). After the cells were cultured, the supernatant of each well was collected, and the amount of newly synthesized collagen in the supernatant was analyzed by measuring the amount of procollagen type I C-peptide (PICP) with a kit (Takara, Japan). The measurement results are shown in Table 5 below.

**Table 5**

| Effect of *Magnolia sieboldii* extract on promotion of collagen synthesis | | | |
|---|---|---|---|
| | Flower extract (v/v%) of *Magnolia sieboldii* of Preparation Example 1 | Whole plant extract (v/v%) of *Magnolia sieboldii* of Preparation Example 2 | Collagen production (absorbance at 450 nm) |
| Control Example 3 | - | - | 1.235 |
| Example 13 | 0.01 | - | 1.356 |
| Example 14 | 0.10 | - | 1.458 |
| Example 15 | 1.00 | - | 1.985 |
| Example 16 | 2.00 | - | 2.246 |
| Example 17 | 5.00 | - | 2.865 |
| Example 18 | - | 0.01 | 1.236 |
| Example 19 | - | 0.10 | 1.385 |
| Example 20 | - | 1.00 | 1.754 |
| Example 21 | - | 2.00 | 1.985 |
| Example 22 | - | 5.00 | 2.136 |

As can be seen from the results in Table 5 above, the *Magnolia sieboldii* extract of the present invention showed a great increase in collagen production compared to Control Example 3 which was not treated with anything. This demonstrates that the *Magnolia sieboldii* extract of the present invention has an excellent effect of promoting the production of collagen. Also, as can be seen from the results of Examples 13 to 17 and Example 18 to 22, the *Magnolia sieboldii* extract of the present invention concentration-dependently promoted the production of collagen, and the flower extract of *Magnolia sieboldii* was superior to the whole plant extract of *Magnolia sieboldii* with respect to the promotion of collagen production as in the case of Experimental Example 4.

The flower extract of *Magnolia sieboldii,* obtained in Preparation Example 1, was used in the following Formulation Examples, but the whole plant extract of *Magnolia sieboldii,* obtained in Preparation Example 2, may be used.

### Formulation Example 1: Skin lotion

Among cosmetic compositions containing the *Magnolia sieboldii* extract, a skin lotion formulation is shown in Table 6 below.

**Table 6**

| ingredients | Content (wt%) |
|---|---|
| Flower extract of *Mugnolia sieboldii,* obtained in Preparation Example 1 | 0.5 |
| 1,3-butylene glycol | 5.2 |
| Oleyl alcohol | 1.5 |
| Ethanol | 3.2 |
| Polysorbate 20 | 3.2 |
| Benzophenone-9 | 2.0 |
| Carboxyvinyl polymer | 1.0 |
| Glycerin | 3.5 |
| Perfume | q.s |
| Preservative | q.s |
| Purified water | Balance |
| Total | 100 |

### Formulation Example 2: Milk lotion

Among cosmetic compositions containing the *Magnolia sieboldii* extract, a milk lotion formulation is shown in Table 7 below.

**Table 7**

| ingredients | Content (wt%) |
|---|---|
| Flower extract of *Magnolia sieboldii,* obtained in Preparation Example 1 | 0.6 |
| Glycerin | 5.1 |
| Propylene glycol | 4.2 |
| Tocopherol acetate | 3.0 |
| Liquid paraffin | 4.6 |
| Triethanolamine | 1.0 |
| Squalane | 3.1 |
| Macadamia nut oil | 2.5 |
| Polysorbate 60 | 1.6 |
| Sorbitan sesquioleate | 1.6 |
| Propylparaben | 0.6 |
| Carboxyvinyl polymer | 1.5 |
| Perfume | q.s |
| Preservative | q.s |
| Purified water | Balance |
| Total | 100 |

### Formulation Example 3: Nutrition cream

Among cosmetic compositions containing the *Magnolia sieboldii* extract, a nutrition cream formulation is shown in Table 8 below.

**Table 8**

| ingredients | Content (wt%) |
|---|---|
| Flower extract of *Magnolia sieboldii,* obtained in Preparation Example 1 | 1.0 |
| Glycerin | 4.0 |
| Vaseline | 3.5 |
| Triethanolamine | 2.1 |
| Liquid paraffin | 5.3 |
| Squalane | 3.0 |
| Beewax | 2.6 |
| Tocopherol acetate | 5.4 |
| Polysorbate 60 | 3.2 |
| Carboxyvinyl polymer | 1.0 |
| Sorbitan sesquioleate | 3.1 |
| Perfume | q.s. |
| Preservative | q.s. |
| Purified water | Balance |
| Total | 100 |

### Formulation Example 4: Massage cream

Among cosmetic compositions containing the *Magnolia sieboldii* extract, a massage cream formulation is shown in Table 9 below.

**Table 9**

| ingredients | Content (wt%) |
|---|---|
| Flower extract of *Magnolia sieboldii,* obtained in Preparation Example 1 | 0.5 |
| Glycerin | 4.0 |
| Vaseline | 3.5 |
| Triethanolamine | 0.5 |
| Liquid paraffin | 24.0 |
| Squalane | 3.0 |
| Beewax | 2.1 |
| Tocopherol acetate | 0.1 |
| Polysorbate 60 | 2.4 |
| Carboxyvinyl polymer | 1.0 |
| Sorbitan sesquioleate | 2.3 |
| Perfume | q.s. |
| Preservative | q.s |
| Purified water | Balance |
| Total | 100 |

### Formulation Example 5: Pack

Among cosmetic compositions containing the *Magnolia sieboldii* extract, a pack formulation is shown in Table 10 below.

**Table 10**

| ingredients | Content (wt%) |
|---|---|
| Flower extract of *Magnolia sieboldii,* obtained in Preparation Example 1 | 1.0 |
| Ethyl alcohol | 3.0 |
| EDTA-2Na | 0.02 |
| Propylene glycol | 5.1 |
| Glycerin | 4.5 |
| Carbopol | 1.0 |
| Polyoxide | 0.1 |
| Preservative | q.s. |
| Perfume | q.s. |
| Purified water | q.s. |
| Total | 100 |

### Experimental Example 6: Effect on protection of skin from UV lights

In order to whether the inventive cosmetic composition containing the *Magnolia sieboldii* extract of the present invention has an effect from protecting the skin from UV lights, the following test was carried out. First, the human skin was artificially irradiated with UV lights (1.5 MED), and then the nutrition cream, prepared in Formulation Example 3, was applied to the human skin at 6-hr intervals. As a comparative example, a composition, which was the same as that of Formulation Example 3, but contained no *Magnolia sieboldii* extract, was used. The test was carried out on 20 persons, and the production of erythema was analyzed through visual evaluation and instrumental evaluation at 48 hours after the UV-light irradiation. The instrumental evaluation was carried out using a skin colorimeter (Minolta, Japan), and the evaluation results are shown in Table 11 below.

**Table 11**

| | Production of erythema | | | |
|---|---|---|---|---|
| | Cream of Comparative Example | | Cream of Formulation Example 3 | |
| | Before use* | After use** | Before use* | After use** |
| Visual evaluation | 0 person | 19 persons | 0 person | 5 persons |
| Instrumental evaluation (a value***) | 5.5 | 16.8 | 5.5 | 8.5 |

| | | | | |
|---|---|---|---|---|
| *before use: before irradiation with UV lights; **after use: 48 hour after irradiation with UV lights; ***a value: indicates the production of erythema on the skin and is expressed as an average value for 20 persons. | | | | |

As can be seen in the results in Table 11 above, the formulation containing the *Magnolia sieboldii* extract of the present invention showed a remarkable reduction in the production of erythema compared to the formulation containing no *Magnolia sieboldii* extract. This suggests that the *Magnolia sieboldii* extract of the present invention has a very excellent effect of inhibiting skin damage caused by UV lights.

### Experimental Example 7: E al valuation of the skin wrinkle reducing effect of cosmetic composition containing Magnolia sieboldii extract

The skin wrinkle reducing effect of the inventive cosmetic composition was evaluated through an actual-use test. In the test, the nutrition cream of Formulation Example 3 containing 1% (v/v) of the *Magnolia sieboldii* extract of Preparation Example 1, and cream, obtained by replacing the *Magnolia sieboldii* extract in the nutrition cream of Formulation Example 3 with purified water, were used. The test was carried out on 30 women, the nourishing cream of Formulation Example 3 was applied to one side of each test subject, and the cream of Comparative Example was applied to the other side. After 6 weeks, the degree of reduction of wrinkles on the surface of each test subject was visually evaluated by professional panels, and the evaluation results are shown in Table 12 below.

**Table 12**

| Wrinkle reducing effect of nutrition cream containing *Magnolia sieboldii* extract (visual evaluation) | | | | |
|---|---|---|---|---|
| Tested cream | skin wrinkle reducing effect | | | Effective rate (%) |
| | Excellent | Moderate | Poor | |
| Cream of Comparative Example | 2 | 2 | 26 | 13.3 |
| Cream of Formulation Example 3 | 23 | 3 | 4 | 86.6 |

As can be seen in Table 12 above, the nutrition cream of Comparative Example 3 containing the *Magnolia sieboldii* extract of the present invention showed an excellent effect of reducing wrinkles, compared to the cream of Comparative Example containing no *Magnolia sieboldii.* Also, no skin irritation could be observed in most of the test subjects whose skin was applied with the inventive cosmetic composition.

## Claims

1. A composition for use in the prevention of photoaging containing an extract of the flower of *Magnolia sieboldii* as an active ingredient.

2. The composition for use as defined in claim 1, wherein the extract of the flower of *Magnolia sieboldii* serves to suppress skin cell damage caused by UV lights and protect skin cell DNA from UV lights, thus protecting the skin and reducing skin wrinkles.

3. The composition for use as defined in claim 1 or 2, wherein the extract of the flower of *Magnolia sieboldii* is contained in an amount of 0.0001-20.0 wt%, to the total weight of the composition.

4. The composition for use as defined in claim 3, wherein the extract of the flower of *Magnolia sieboldii* is contained in an amount of 0.01-10.0 wt%, to the total weight of the composition.

5. The composition for use as defined in any one of claims 1 to 4, wherein the composition is in the form of a formulation selected from the group consisting of solution, suspension, emulsion, paste, gel, cream, lotion, soap, surfactant-containing cleansing oil, powder foundation, emulsion foundation, wax foundation and spray.

6. A make-up method for reducing skin wrinkles, comprising applying to the human skin a cosmetic composition containing an extract of the flower of *Magnolia sieboldii* as an active ingredient such as defined in any one of claims 1 to 5.

## Patentansprüche

1. Zusammensetzung zur Verwendung bei der Prävention der Lichtalterung, die einen Extrakt der Blüte von *Magnolia sieboldii* als Wirkstoff enthält.

2. Zusammensetzung zur Verwendung gemäß Anspruch 1, wobei der Extrakt der Blüte von *Magnolia sieboldii* dazu dient, Hautzellschäden durch UV-Licht zu unterdrücken und die DNA der Hautzellen vor UV-Licht zu schützen, so dass die Haut geschützt wird und Hautfalten reduziert werden.

3. Zusammensetzung zur Verwendung gemäß Anspruch 1 oder 2, wobei der Extrakt der Blüte von *Magnolia sieboldii* in einer Menge von 0,0001 bis 20,0 Gew.-%, bezogen auf das Gesamtgewicht der Zusammensetzung, enthalten ist.

4. Zusammensetzung zur Verwendung gemäß Anspruch 3, wobei der Extrakt der Blüte von *Magnolia sieboldii* in einer Menge von 0,01 bis 10,0 Gew.-%, bezogen auf das Gesamtgewicht der Zusammensetzung, enthalten ist.

5. Zusammensetzung zur Verwendung gemäß einem der Ansprüche 1 bis 4, wobei die Zusammensetzung in Form einer Zubereitung vorliegt, die aus der Gruppe ausgewählt ist, die aus einer Lösung, Suspension, Emulsion, Paste, einem Gel, einer Creme, Lotion, Seife, einem tensidhaltigen Reinigungsöl, einer Pulver-Foundation, Emulsions-Foundation, Wachsfoundation und einem Spray besteht.

6. Make-Up-Verfahren zum Reduzieren von Hautfalten, umfassend das Auftragen einer Kosmetikzusammensetzung, die einen Extrakt der Blüte von *Magnolia sieboldii* als Wirkstoff enthält, wie sie in einem der Ansprüche 1 bis 5 definiert ist, auf die menschliche Haut.

## Revendications

1. Composition pour une utilisation dans la prévention du photovieillissement, contenant un extrait de la fleur de *Magnolia sieboldii* comme principe actif.

2. Composition selon la revendication 1, dans laquelle l'extrait de la fleur de *Magnolia sieboldii* sert à supprimer les détériorations des cellules de la peau causées par les rayons ultraviolets et à protéger l'ADN des cellules cutanées contre les rayons ultraviolets, protégeant ainsi la peau et atténuant les rides.

3. Composition selon la revendication 1 ou la revendication 2, dans laquelle l'extrait de la fleur de *Magnolia sieboldiï* est présent à raison de 0,0001-20,0% en poids, par rapport au poids total de la composition.

4. Composition selon la revendication 3, dans laquelle l'extrait de la fleur de *Magnolia sieboldii* est présent à raison de 0,01-10,0% en poids, par rapport au poids total de la composition.

5. Composition selon l'une quelconque des revendications 1 à 4, ladite composition étant sous la forme d'une formulation choisie dans le groupe constitué par une solution, une suspension, une émulsion, une pâte, un gel, une crème, une lotion, un savon, une huile nettoyante contenant un agent tensioactif, un fond de teint en poudre, un fond de teint en émulsion, un fond de teint cire et un spray.

6. Procédé de maquillage permettant d'atténuer les rides de la peau, comprenant l'application sur la peau humaine d'une composition cosmétique contenant un extrait de la fleur de *Magnolia sieboldiï* comme principe actif, telle que définie dans l'une quelconque des revendications 1 à 5.
